# EUROPEAN PATENT APPLICATION

(11) **EP 1 695 732 A1**
(43) Date of publication of application: **30.08.2006**
(21) Application number: 06003973.2
(22) Date of filing: 27.02.2006
(51) Int. Cl.: A61M 16/20, A61M 16/06, F16K 31/08, F16K 17/02, A62B 9/02

(54) **Pressure relief valve**

(30) Priority: 28.02.2005 NZ 53855905
(71) Applicant: Fisher & Paykel Healthcare Limited, East Tamaki, Auckland (NZ)
(72) Inventor: Van Beurden, Jason Peter, New Lynn Auckland (NZ); Orton, Reginald James McKenzie, Greenhithe Auckland (NZ)
(74) Representative: Hoarton, Lloyd Douglas Charles

(57) **Abstract**

A pressure relief valve is disclosed that may be used as both an over pressure control valve, preventing possible barotraumas to the patient; or as an overpressure control valve for a breathing circuit preventing over pressures caused by blockages in a breathing circuit (supplying breathing gases to a patient) with the potential to damage either the humidifier or the breathing circuit. The pressure relief valve (100) comprises a magnetic seating (101) that is associated with an outlet vent (104) formed on a breathing assistance apparatus (2), and a magnetic cover (102) capable of covering the seating and the outlet vent. The cover is held against the seating by a magnetic force between the cover and the seating. The outlet vent is substantially sealed by the cover and when the pressure of the gases in the breathing assistance apparatus exceeds a predetermined pressure value that overcomes the magnetic force, the cover releases from the seating to allow gases to exit the outlet vent and reduce the gases pressure in the breathing assistance apparatus.

## Description

### FIELD OF INVENTION

This invention relates to pressure relief valves and particularly to pressure relief valves for use in a positive pressure ventilation system and other breathing assistance apparatus.

### SUMMARY OF THE PRIOR ART

Existing devices for reducing the pressure in such breathing assistance apparatus as nasal cannula, masks or the like for the provision of a gases supply (and most particularly positive pressure gases supply) to a patient have various disadvantages.

Bias flow holes are one way in which pressure in apparatus such as a mask, nasal cannula or the like has been relieved. However, these are intended as a general pressure reduction system to supply an "on-demand" flow to the patient. Thus pressure reduction will occur throughout all the pressure ranges and will not maintain much pressure at low levels of flow of gases to the patient.

Over pressure relief valves operating with a spring or the elastic deformation of parts exist. However, the major disadvantages to these devices are that such valves start venting pressure as soon as there is any positive pressure within the apparatus, gradually increasing the venting as the pressure increases. Furthermore, the spring force often continues to act within the valve and as a consequence the delivered pressure continues to climb as the supplied pressure is increased. In this case usually not all respiratory gases are delivered at targeted normal operating pressures.

In other industries relief valves also exist. These valves are generally used in the petrochemical industries as large fluid relief valves for storage tanks, chemical refining plants and other such heavy industry uses.

Instrumentation Industries produce a magnetic pressure control (PEEP) valve (United States Patent Number 4,210,174). This valve has a valve member with a central magnetically attracted member spaced from and coaxial with rod like magnet. The magnet and valve member are relatively movable to adjust the magnetic attraction between them and thus the opening pressure of the valve. This valve operates on the basis that the magnet will work as a constant force spring when placed at a set distance from ferromagnetic material. These pressure control valves are designed to provide a constant pressure as set by the user, rather than as a safety valve.

### SUMMARY OF THE INVENTION

It is an object of the present invention to provide a valve for a breathing assistance apparatus that will obviate the above disadvantages or will at least provide healthcare providers with a useful choice.

Accordingly in the first aspect the present invention consists in a pressure relief valve for use with a breathing assistance apparatus for delivering pressurised gases to a patient by way of a gases transport means comprising:
a magnetic seating about.or in association with an outlet vent on said breathing assistance apparatus,
a magnetic cover capable of covering said seating and said outlet vent,
wherein during normal use said cover is held against said seating by a magnetic force between said cover and said seating such that said outlet vent is substantially sealed by said cover, and
wherein when the pressure of said gases in said breathing assistance apparatus exceeds a predetermined value said magnetic force is overcome by said pressure of said gases at said predetermined value releasing said cover from said seating to allow gases to exit said outlet vent and reduce the gases pressure in said breathing assistance apparatus.

Preferably said breathing assistance apparatus is a nasal cannula.

Preferably said nasal cannula comprises:
a face mount part, including at least one nasal prong capable of fitting into at least one of said patient's nares and,
a gases flow manifold part in fluid communication with said face mount part; said manifold part having a single horizontal side gases entry, in use, in fluid communication with said transport means.

Preferably said outlet vent is formed on said gases flow manifold part and said magnetic seating is positioned about said vent.

Alternatively said breathing apparatus is a humidifier with a gases inlet and a gases outlet and said valve is positioned on one of said inlet and outlet.

Alternatively said breathing assistance apparatus is a face mask.

Preferably said gases supplied to said face mask are humidified by a humidifier and said magnetic seating and said magnetic cover are constructed on an inlet to said humidifier.

Preferably said magnetic seating is a ring of ferrite impregnated plastic material.

Preferably said magnetic cover is constructed from a ferromagnetic material.

Alternatively said cover is constructed from a magnetised material of opposition polarity to the polarity of said magnetic seating.

Preferably said magnetic cover is hingedly arranged in association with said magnetic seating.

### BRIEF DESCRIPTION OF THE DRAWINGS

Preferred forms of the present invention wih now be described with reference to the accompanying drawings.
Figure 1 is an illustration of a respiratory humidification system and nasal cannula capable of use with a pressure relief valve of the present invention.
Figure 2 is a perspective view of a first embodiment of a pressure relief valve of the present invention as used on a nasal cannula, where the valve is in a closed position.
Figure 3 is a perspective view of a pressure relief valve of Figure 2, where the valve is in an open position.
Figure 4 is an exploded view of the valve of Figure 2.
Figure 5 is a second embodiment of the valve of the present invention where the valve has a cover that is constrained by a cage and spring.
**Figure 6** is a third embodiment of the valve of the present invention where the valve is of a mechanical hinge type valve, and is shown in an open position.
**Figure 7** is a forth embodiment of a valve of the present invention where the valve has a valve cover constrained by a plunger system.
**Figure 8** is a side view of the fifth embodiment of the valve of the present invention where the valve is a pressure pop off valve as may be used at an inlet or outlet of a humidification chamber.
Figure 9 is a perspective view of the valve of Figure 8.
Figure 10 is a graph showing pressure versus flow for the pressure relief valve of the first embodiment of the present invention and of a prior art spring valve.
Figure 11 is an illustration of an alternative embodiment of a respiratory humidification system and face mask with a pressure relief valve of the present invention on the humidifier inlet.

### DETAILED DESCRIPTION OF THE PREFERRED EMBODIMENTS

The pressure relief valve of the present invention may be used as both an over pressure control valve, preventing possible barotraumas to the patient; or as an overpressure control valve for a breathing circuit preventing over pressures caused by blockages in a breathing circuit (supplying breathing gases to a patient) with the potential to damage either the humidifier or the breathing circuit.

### Breathing Assistance Apparatus

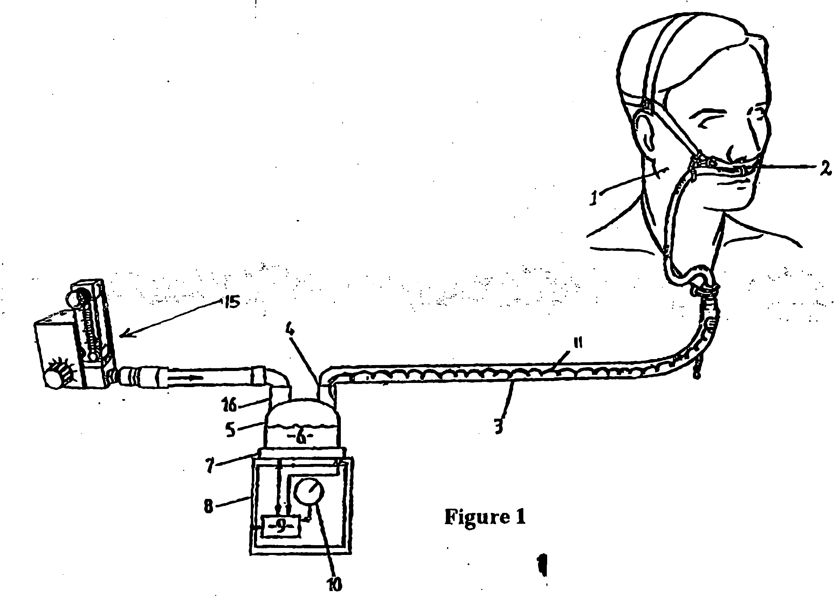

Referring to Figure 1 a breathing assistance apparatus including a humidifier as might be used with the nasal cannula and a pressure relief valve of the present invention is shown. A patient 1 is receiving humidified and pressurised gases though a nasal cannula 2 connected to a humidified gases transportation pathway or inspiratory conduit 3. The conduit 3 is connected to a humidifier 8 (including humidification chamber 5) that is supplied with gases from a gases supply, such as a gases blender 15 or other appropriate gases supply means, such as a blower or continuous positive pressure device. The inspiratory conduit 3 is connected to the outlet 4 of a humidification chamber 5 that contains a volume of water 6. The humidification chamber 5 is preferably formed from a plastics material and may have a highly heat conductive base (for example an aluminium base) which is in direct contact with a heater plate 7 of the humidifier 8. The humidifier 8 is provided with control means or electronic controller 9 that may comprise a microprocessor based controller executing computer software commands stored in associated memory, Gases flowing through the inspiratory conduit 3 are passed to the patient by way of the nasal cannula 2.

The controller 9 receives input from sources such as user input means or dial 10 through which a user of the device may, for example, set a predetermined required value (preset value) of humidity or temperature of the gases supplied to patient 1. In response to the user set humidity or temperature value input via dial 10 and other possible inputs such as internal sensors that sense gases flow or temperature, or by parameters calculated in the controller, controller 9 determines when (or to what level) to energise heater plate 7 to heat the water 6 within humidification chamber 5. As the volume of water 6 within humidification chamber 5 is heated, water vapour begins to fill the volume of the chamber above the water's surface and is passed out of the humidification chamber 5 outlet 4 with the flow of gases (for example air) provided from a gases supply means or blender 15 which enters the chamber through inlet 16. It should be noted that it is possible to obtain the relationship between the humidity of the gases in humidification chamber 5 and the temperature of the heater plate 7. Accordingly, it is possible to utilise the heater plate temperature in an algorithm or a look-up table to determine the humidity of the gases.

It is preferred that an oxygen blender is provided with the breathing assistance apparatus of the present invention, although other gases supply apparatus may be used. The oxygenblender 15 as shown in Figure 1 supplies a blend of oxygen and air, or any other medical gases, that are supplied from a compressed medical gases line. The gases line is usually of a pressure approximately 6 bar and supplied at a hospital or the like.

A heating element 11 may be provided within the conduit or tubing 3 to help prevent condensation of the humidified gases within the conduit. Such condensation is due to the temperature of the walls of the conduit being close to the ambient temperature, (being the temperature of the surrounding atmosphere) which is usually lower than the temperature of the humidified gases within the conduit. The heater element effectively replaces the energy lost from the gases through conduction and convection during transit through the conduit. Thus the conduit heater element ensures the gases delivered are at an optimal temperature and humidity.

### Nasal Cannula

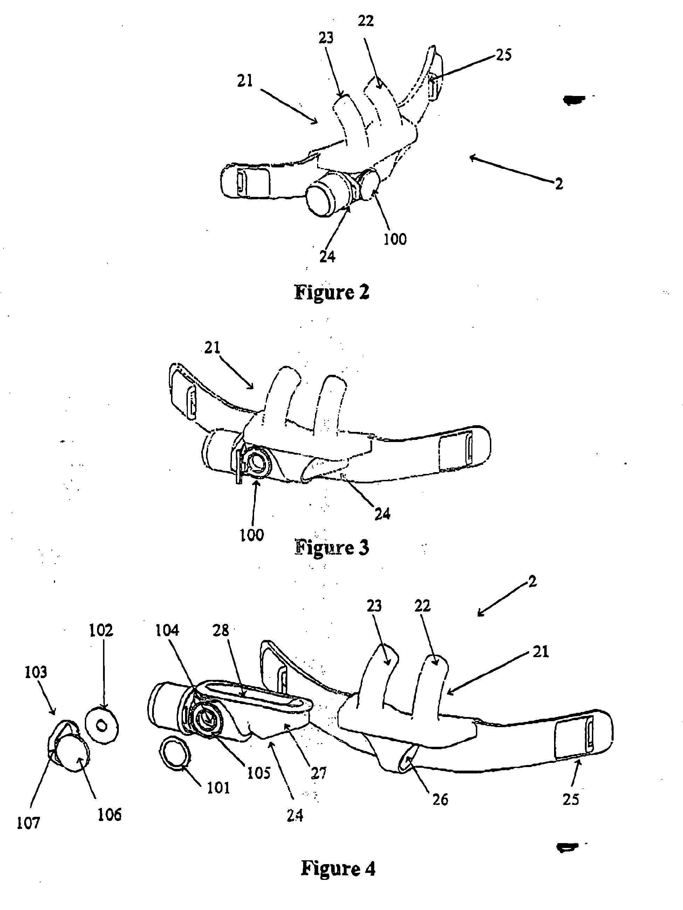

Referring to Figures 2 to 4, the pressure relief valve 100 in a first embodiment is shown on a nasal cannula 2. The nasal cannula is of the type described in New Zealand Patent Application No. 526362 of Fisher & Paykel Healthcare Limited and also in United States Patent Application Number 10/855,146, the contents of which are incorporated herein.

The nasal cannula 2 has a face mount part 21, a pair of nasal prongs 22, 23 and gases flow manifold part 24 that attaches to the inspiratory conduit 3. The face mount part 21 and pair of nasal prongs 22, 23 are preferably integrally molded as one piece from a soft plastics material such as silicone, although in other forms the face mount part and prongs may be two parts that are fixed to one another in use. The nasal prongs 22, 23 are tubular in shape and maybe consistent in diameter but may be shaped to fit the contours of the human nares.

A strap or strap attachment means 25 may be integrally formed or attached to the face mount part 21 in orderto enable the nasal cannula assembly 2 to be held in place about apatient's face.

The face mount part 21 has an open tubular recess 26 extending below the nasal prongs 22, 23 that is capable of receiving a gases flow manifold part 24 that is attached to or integrally formed with the inspiratory conduit 3. The tubular passageways within the nasal prongs 22, 23 extend through the face mount part 21 and into the recess 26. The gases flow manifold part 24 is blocked at one end 27 but attached to the conduit at the other end and has an elongate opening 28 that acts as an exit for gases received from the conduit 3. Due to the flexible nature of the material the face mount part 21 is made from, and as the gases flow manifold part 24 is made from a hard plastics material, the gases flow manifold part 24 can be pushed through the tubular recess 26 in the face mount part 21. Thus, the elongate opening 28 in the gases flow manifold part 24 meets with the tubular passageways of the prongs 22, 23. Therefore, in use, gases flowing through the conduit 3 and into the gases flow manifold part 24 exit through the opening 28 and into the tubular passageways in the prongs 22,23, then into the patient's nares.

### Pressure Relief Valve

The pressure relief valve of the present invention is predominantly for use with a breathing assistance apparatus that is capable of delivering pressurised gases to a patient by way of a gases transport means (conduit or tubing, as described above). The pressure relief valve of the present invention has a magnetic seating about or in association with an outlet vent on the breathing assistance apparatus and a magnetic cover capable of covering the seating and the outlet vent. During normal use the magnetic cover is held against the magnetic seating by a magnetic force between the cover and the seating such that the outlet vent is substantially sealed by the cover. When the pressure of the gases through said breathing assistance apparatus exceeds a predetermined pressure value the magnetic force between the cover and seating is overcome by the pressure of the gases in the breathing assistance apparatus. Consequently, the cover is released from the seating and the outler vent opens to allow gases to exit the outlet vent and reduce the gases pressure within the breathing assistance apparatus.

The pressure relief valve 100 of the present invention consists of a magnetised valve seat 101 that is sealed with a ferromagnetic cover 102, constrained by a valve cover 103. The valve operates by the sealing of the valve seat 101 and ferromagnetic cover 102 together, until a point where the internal pressure is greater than the magnetic sealing between the seating 101 and the ferromagnetic cover 102. When the internal pressure is greater than the magnetic sealing the valve 100 opens and a portion of the flow of gases through the cannula is vented and the pressure in the cannula 2 is lowered to a safe level.

Referring to Figure 4, in the preferred form of the breathing assistance of the present invention, an aperture 104 is formed in the nasal cannula manifold 24 with a circular recess 105 formed in the manifold 24 to house the magnetic valve seating 101. The aperture 104 acts as an outlet vent for exhaust gases from the breathing assistance apparatus. The magnetic valve seating 101 is preferably a ring constructed from ferrite or alternatively a ferrite impregnated plastic or any other appropriate material, such as stainless steel that has been magnetized. The valve seating 101 is separated from the gas stream, but flush with the surface of the nasal cannula manifold 24, therefore, it is housed in the circular recess 105. The ferromagnetic cover 102, also preferably circular in shape, is attached to the valve cover 103 and in a valve closed position it is seated flush and substantially airtight with the magnetic valve seating 101.

The valve cover 103 is comprised of a thermoplastic flap 106 that is hinged to a manifold attachment 107. The manifold attachment 107 in this embodiment of the present invention is a ring that can be slid about the tubing part of the nasal cannula manifold 24. Other suitable attachment mechanisms between the manifold and cover are described in more detail below.

The valve cover 103 is hinged around a point to the side of the valve seating 101, such that the valve cover opens in a manner similar to a hatch or trap door as can be seen in Figure 3. The seal between the ferromagnetic cover 102 and the valve seating 101 is maintained by the magnetic attraction between the materials these two parts of constructed of Due to the exponential relationship between the proximity of these parts and the force generated, the valve opens rapidly once the magnetic force of the closed valve is overcome. The magnetic force exerted on the flap 106 is greatly reduced once the valve 100 opens; enabling the gases pressure behind the valve 100 to open it fully. Consequently, the pressure inside the cannula 2 will drop to a lower level than that before the valve 100 had opened, The initial seating force between the valve seating 101 and ferromagnetic cover 102 allows the pressure to normally fluctuate inside the cannula 2 when delivering positive pressure ventilation by way of high flow nasal cannula 2 while the valve 100 is closed. However, if the pressure reaches an unsafe level, the valve 100 will open, venting the majority of the gases pressure causing the pressure in the cannula to reduce to a safe and.low level. The unsafe level of pressure within the breathing assistance apparatus is preferably predetermined by testing and therefore the magnetic valve and magnetic sealing level (as previously described) can be designed and material structures altered depending on the pressure level required to be set by the manufacturer or user.

Once the pressure within the breathing assistance apparatus drops in use below a threshold reseating pressure associated with the valve (and predetermined by the elasticity of the elastic hinge in the valve cover 103), the valve flap 106 will close, due to the elastic hinge in the valve cover 103 between the manifold attachment 107 and flap 106. The valve will then seal again due to the magnetic attraction between the ferromagnetic cover 102 and valve seating 101 and automatically reset, on the assumption that whatever caused the aforementioned pressure build up has been relieved.

The aperture 104 size is such that when the valve 100 has opened, the pressure through the cannula 2, in a manner similar to a bias flow outlet vent, remains sufficient to deliver an adequate flow to the patient 1.

### Advantages

The pressure relief valve 100 of the present invention has the ability to maintain 100% pressure delivery, providing therapeutic benefits of positive pressure ventilation, up until the point where the valve opens due to an unsafe pressure, at which time the pressure is lowered significantly, in order to avoid injury to the patient.

Positive pressure ventilation to patients by high flow nasal cannula requires that the cannula does not seal against the nares, as the high flow and the small gap between the nares and the cannula induce this pressure. If for some reason the nasal cannula does seal against the nares of the patient, and the patient also has an air-tight seal around the lips, the pressure within the cannula might build up to a level high enough to induce barotraumas, resulting in patient injury or, in highly unlikely and extreme cases, death. As a consequence, the pressure relief valve 100 of the present invention is preferably placed in parallel with the nasal cannula 2, which if pressures reach a set level (that arc considered to be unsafe) will open, significantly reducing the pressure delivered to the patient

The use of a magnetic seal in a pressure relief valve as described above allows the construction of a valve to be very simple requiring minimal parts. The magnetic pressure relief valve of the present invention consists effectively of only three parts, therefore is relatively low cost.

Existing valves with springs require complicated assembly, and often calibration on line, causing excessive cost. The pop-off pressure of the valve is a function of the materials used and the dimensions of the valve parts; thus the valve behaviour can be modified to suit different pressures/applications. The reseating pressure of the valve is a function of both the magnetic attraction force between the magnetic cover and the magnetic seating, and also of the elastic properties of the hinging mechanism. Therefore, altering the hinging mechanism will alter the characteristics of the valve.

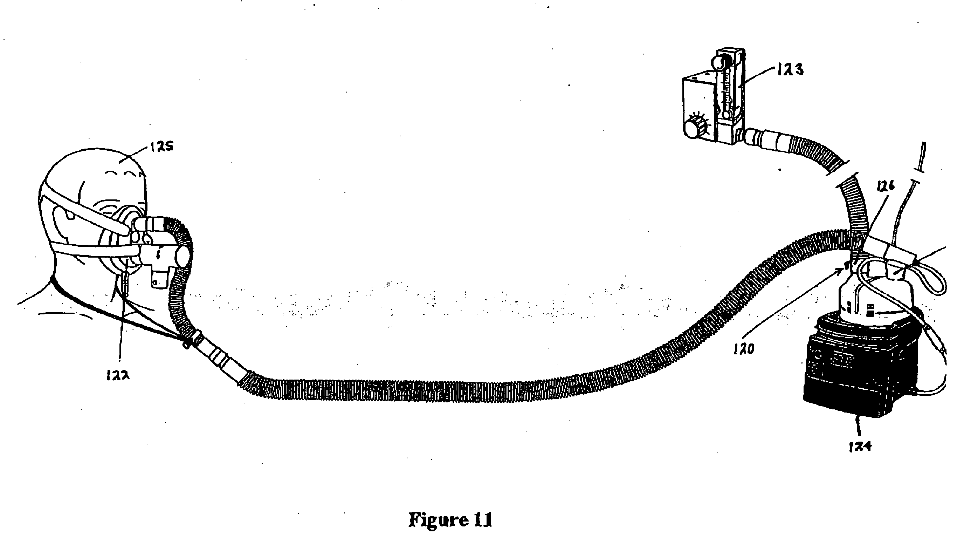

Figure 10 shows a graph of the pressure versus the leak flow for both a spring type pressure relief valve and the magnetic pressure relief valve of the present invention. As can be seen, when the magnetic valve reaches its break open pressure (see A on Figure 10) the pressure inside the nasal cannula drops, allowing a large amount of gases to be vented before the pressure may rise again to a high level. The spring valve, upon breaking, only just starts to vent a small amount of gases. In order for a spring valve to vent a large amount of air, the pressure inside the cannula that is being delivered to the patient, will increase to a higher level than that at which the valve started to open.

The graph in Figure 10 shows a discontinuity when the pressure breaks at approximately 12cm pressure for the pressure relief valve of the present invention. This discontinuity shows that as the pressure reaches 12cm H₂O of pressure, the valve opens and vents air such that the pressure drops almost instantaneously to about 2cm H₂O, and the leak flow increases to approximately 14L/min, As the pressure increases, the flow through the valve increases rapidly. Conversely, the spring valve shows no discontinuity, and once the spring valve opens, at around 10cm H₂O, the pressure continues to climb rapidly as more flow is introduced,

Because of the characteristics mentioned above, the pressure relief valve of the present invention is able to vent large amounts of gas, without delivering a high and possibly dangerous pressure to the patient. This is a major advancement over prior art valves. Also the ability to maintain a leak free seal up until the point where the pressure becomes dangerous is another benefit of the valve of the present invention.

Finally, the pressure relief valve of the present invention has small dimensions and is lightweight providing further advantages over prior art valves.

### Alternative Valve Cover Embodiments

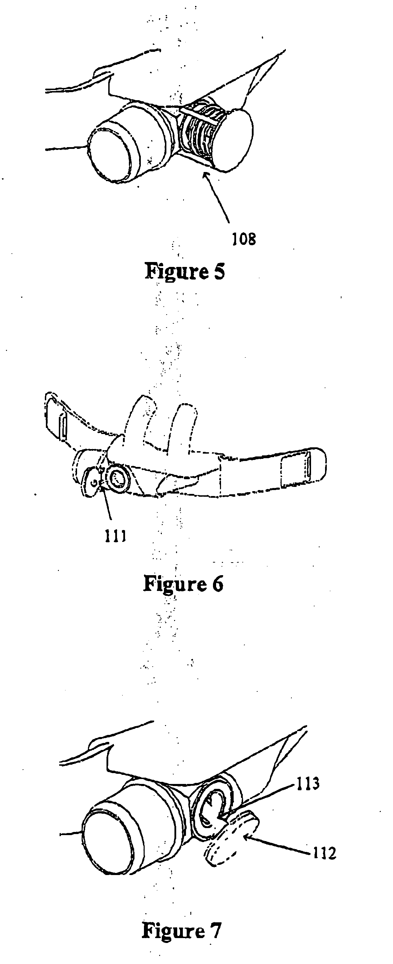

The ferromagnetic cover 102 may be constrained in a number of ways. It is proposed that the cover 102 to be attached via a thermoplastic hinge (as shown in Figures 2 to 4), or alternately constrained via a cage 108 (as shown in Figure 5) and controlled in a directional manner.

The use of a thermoplastic (or similar) hinge is highly suitable for integration into the end of a nasal cannula near the patient as it has the advantage of potentially being light weight. The cage design, due to its larger physical dimensions imposed by the required construction, might be more suitable for use as an over pressure safety valve at the gas supply (blender 15) or humidification chamber (inlet or outlet).

Other variations for constraining the ferromagnetic valve cover 102 include:
- capturing the valve seating (not shown) within a cage 108, with the ferromagnetic valve cover 110 guided by at least one pin 109 (see Figure 5),
- constraining the valve cover 112 by a tie rod 113 as shown in Figure 7, this embodiment would require a manual reseating pressure to be applied, either by the patient or a caregiver, or
- use of a multi element traditional hinge element 111 (see Figure 6), or anything that can be used to provide a similar result.

### Chamber Valve

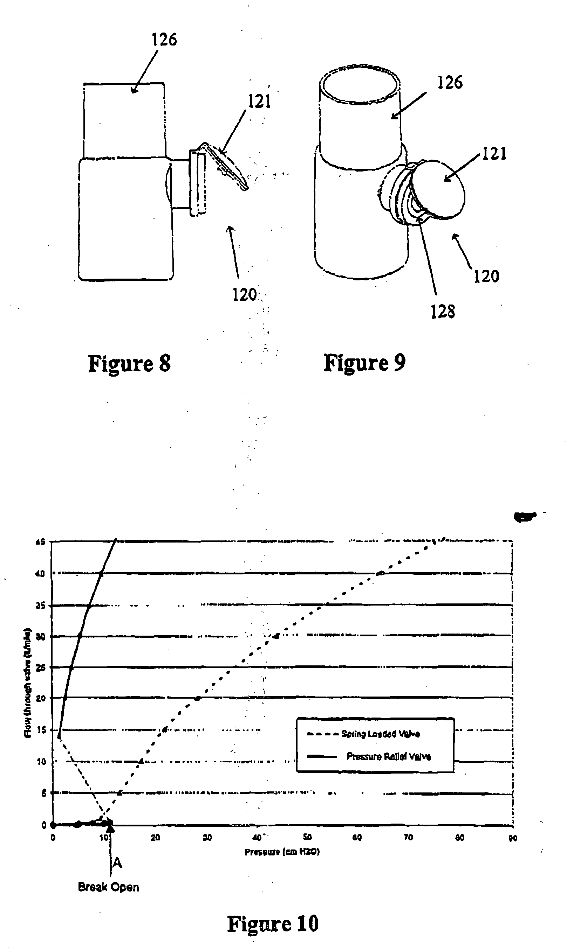

A fourth embodiment of the valve of the present invention is shown in Figures 8, 9 and 11. Here the pressure relief valve 120 is constructed on a humidification chamber 5 inlet 16 or outlet 4 of a humidifier 8. The form of the valve 120 is preferably of the construction described above in relation to Figure 2, with a valve seating 128 and valve cover 121. In this embodiment a slightly bulkier, more robust design would be possible, while still operating on the same principle. This embodiment of the valve may have the advantage that it can also prevent against pressure within the complete apparatus, gases supply, humidifier and conduits, caused by a blockage or obstruction, which may have the potential to cause harm to any equipment that is exposed to this pressure.

In figure 11 the patient interface 122 is a full face mask and gases are supplied at a high pressure by an oxygen blender 123 to a humidifier 124 then to the patient 125. In this embodiment is it recommended that a relief valve 120 is provided at the inlet 126 (or outlet 127) to the humidifier.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A pressure relief valve (100) for use with a breathing assistance apparatus for delivering pressurised gases to a patient by way of a gases transport means (3) comprising:
a magnetic seating (101) about or in association with an outlet vent (104) on said breathing assistance apparatus (2),
a magnetic cover (102) capable of covering said seating (101) and said outlet vent (104),
wherein during normal use said cover (102) is held against said seating (101) by a magnetic force between said cover and said seating such that said outlet vent is substantially sealed by said cover, and
wherein when the pressure of said gases in said breathing assistance apparatus (2) exceeds a predetermined value said magnetic force is overcome by said pressure of said gases at said predetermined value releasing said cover from said seating (101) to allow gases to exit said outlet vent (104) and reduce the gases pressure in said breathing assistance apparatus (2).

2. A pressure relief valve for use with a breathing assistance apparatus according to claim 1 wherein said breathing assistance apparatus is a nasal cannula (2).

3. A pressure relief valve for use with a breathing assistance apparatus according to claim 2 wherein said nasal cannula comprises:
a face mount part (21), including at least one nasal prong (22, 23) capable of fitting into at least one of said patient's nares and,
a gases flow manifold part (24) in fluid communication with said face mount part (21), said manifold part (24) having a single horizontal side gases entry, in use, in fluid communication with said gases transport means (3).

4. A pressure relief valve for use with a breathing assistance apparatus according to claim 3 wherein said outlet vent (104) is formed on said gases flow manifold part (24) and said magnetic seating (101) is positioned about said vent (104).

5. A pressure relief valve for use with a breaching assistance apparatus according to claim 1 wherein said breathing assistance apparatus is a humidifier (8) with a gases inlet (16) and a gases outlet (4) and said valve (100) is positional on one of said inlet and outlet.

6. A pressure relief valve for use with a breaming assistance apparatus according to claim 1 wherein said breathing assistance apparatus is a face mask (122).

7. A pressure relief valve for use with a breathing assistance apparatus according to claim 6 wherein said gases supplied to said face mask (122) are humidified by a humidifier (124) and said magnetic seating and said magnetic cover are constructed on an inlet (126) to said humidifier.

8. A pressure relief valve for use with a breathing assistance apparatus according to any one of claims 1 to 7 wherein said magnetic seating (101) is a ring of ferrite impregnated plastic material.

9. A pressure relief valve for use with a breathing assistance apparatus according to claim 8 wherein said magnetic cover (102) is constructed from a ferromagnetic material.

10. A pressure relief valve for use with a breathing assistance apparatus according to any one of claims 1 to 9 wherein said cover is constructed from a magnetised material of opposite polarity to the polarity of said seating.

11. A pressure relief valve for use with a breathing assistance apparatus according to any one of claims 1 to 10 wherein said cover is hingedly arranged in association with said seating.
